# EUROPÄISCHE PATENTSCHRIFT

(11) **EP 1 603 498 B1**
(45) Veröffentlichungstag und Bekanntmachung des Hinweises auf die Patenterteilung: **20.08.2008**
(21) Anmeldenummer: 04715866.2
(22) Anmeldetag: 01.03.2004
(51) Int. Cl.: A61F 5/058

(54) **VORRICHTUNG ZUR SCHMERZLINDERNDEN IMMOBILISIERUNG VON GEBROCHENEN RIPPEN**
DEVICE FOR THE ANALGESIC IMMOBILISATION OF BROKEN RIBS
DISPOSITIF POUR L'IMMOBILISATION ANTALGIQUE DE COTES CASSEES

(30) Priorität: 03.03.2003 CH 3282003
(43) Veröffentlichungstag der Anmeldung: 14.12.2005
(73) Patentinhaber: Chrisofix AG, 8212 Neuhausen (CH)
(72) Erfinder: BOLLA, Kalman, CH-8212 Neuhausen am Rheinfall (CH)
(74) Vertreter: Kis-Kovacs, Annemarie
(86) Internationale Anmeldenummer: PCT/CH2004/000109
(87) Internationale Veröffentlichungsnummer: WO 2004/078079

(56) Entgegenhaltungen:
- WO-A-89/05620
- US-A- 6 039 706

## Beschreibung

### TECHNISCHES GEBIET

Die vorliegende Erfindung bezieht sich auf das Gebiet der medizinischen Hilfsmittel. Sie betrifft eine Vorrichtung zur schmerzlindernden Immobilisierung von gebrochenen Rippen (Thorax-Immobilisierungsvorrichtung) gemäss dem Oberbegriff des Anspruchs 1.

Eine solche Vorrichtung ist z.B. aus der Druckschrift US-A-4,312,334 bekannt.

### STAND DER TECHNIK

Rippenfrakturen sind, gerade wenn mehrere Rippen gleichzeitig gebrochen sind, sehr schmerzhaft. Die gebrochenen Rippen verlieren die mechanische Stabilität und sind sogar in besonderen Fällen wie z.B. Fensterbrüchen nicht länger in der Lage, den Brustkorb so aufzuspannen, dass die innerhalb des Brustkorbs angeordneten Lungen ungehindert arbeiten können. Dies macht sich insbesondere beim Atmen bemerkbar, das dem Patienten Schmerzen bereitet und in dazu bringt, flach zu atmen (reduzierte forcierte Vitalkapazität FVC) oder (bei mehrfachen Brüchen) sogar in eine paradoxe Atmung zu verfallen, bei der die Bewegungen des an der Atmung beteiligten Brustbereiches den Bewegungen bei der normalen Atmung entgegengesetzt verlaufen. Da in den meisten Fällen bei einer Rippenfraktur kein Eingriff vorgenommen wird, sondern die selbsttätige Heilung abgewartet wird, ist es wünschenswert, in diesem Zeitraum dem Patienten zusätzlich oder sogar ersatzweise zu Medikamenten Erleichterung von den auftretenden Schmerzen zu verschaffen und damit eine Verbesserung der Atmung zu erreichen.

Zur Immobilisierung von gebrochenen Rippen ist es seit langem bekannt, die Seite des Thorax mit der Rippenfraktur mit Pflastern zu verkleben und so eine Reduktion bei der Bewegung der gebrochenen Rippen zu erreichen, die aber ungenügend ist. Es ist auch bereits vorgeschlagen worden (GB-A-626,425), anstelle der Pflaster eine gürtelartige Bandage zu verwenden, die mittels einer lösbaren Spannvorrichtung vorgespannt werden kann. Derartige Immobilisierungsvorrichtungen führen zwar zu einer eingeschränkten Beweglichkeit im Bereich der Rippenfraktur, bewirken jedoch gleichzeitig auch eine massive Beschränkung in der Atmung.

In der eingangs genannten Druckschrift US-A-4,312,334 ist demgegenüber vorgeschlagen worden, dem Patienten ein Gestell umzuschnallen, das auf der Vorderseite im Bereich des Brustkorbes zwei vertikal angeordnete, bogenförmig oberhalb des Brustkorbes verlaufende Stützelemente aufweist. Der im Bereich der Rippenfraktur eingefallene Teil des Thorax wird mittels eines Drahtes nach aussen gezogen, der mit seinem einen Ende am Brustkorb und mit dem anderen Ende am zugehörigen Stützelement befestigt ist. Auf diese Weise können die gebrochenen Rippen in einer für die Heilung geeigneten Position gehalten und die Atmung bzw. die Schmerzen des Patienten erleichtert werden.

Nachteilig ist bei dieser Art der Entlastung einerseits der notwendige Eingriff und hohe Aufwand im Hinblick auf die Befestigung der Drähte und andererseits die durch das Gestell und die gespannten Drähte verursachte Behinderung der Bewegungsfreiheit des Patienten.

Schliesslich ist aus der WO-A1-89/05620, die als nächster Stand der Technik anerkannt wird, eine Fixierungsplatte für Rippenfrakturen bekannt, welche in einer Längsrichtung biegesteif ist, dagegen in einer Richtung senkrecht dazu in einem gewissen Umfang biegsam ist und entlang der Diagonalen in gewissem Umfang verdrehbar (tordierbar) ist. Hierdurch sollen einerseits die einzelnen gebrochenen Rippen unterstützt und fixiert werden, andererseits jedoch die natürliche Bewegung der Rippen beim Atmen ermöglicht werden. Erreicht wird dies durch Verwendung einer Platte aus einem elastisch biegsamen Material wie z.B. Gummi oder Kunststoff, in der parallel zur biegesteifen Längsrichtung verlaufend mehrere geschlossene, längliche Hohlräume angeordnet sind. In jedem dieser Hohlräume ist als 1-dimensionales Schienungselement ein Stab aus einem inelastischen aber deformierbaren Material frei beweglich untergebracht. Im Falle einer Rippenfraktur werden die Stäbe durch entsprechende Deformation an die Kontur der zu schienenden Rippen angepasst. Die Platte mit den angepassten Stäben wird dann flächig auf den Brustkorb aufgeklebt, wobei die Stäbe parallel zu den Rippen verlaufen. Die Rippen sollen so in ihrer Längsrichtung fest fixiert werden, während der Brustkorb sich beim normalen Atmen weitgehend ungehindert aufweiten kann.

Die 1-dimensionale Schienung der gebrochenen Rippen fixiert zwar die Rippen in ihrer Längsrichtung, lässt jedoch im Hinblick auf eine ungehinderte Atmung die Beweglichkeit der Rippen relativ zueinander weitgehend unbehindert bestehen. Dazu trägt insbesondere auch der Umstand bei, dass die Stäbe frei beweglich in den Hohlräumen gelagert sind. Aufgrund dieser relativen Beweglichkeit der Rippen zueinander ändern sich atmungsbedingt die Zwischenräume zwischen den Rippen. Dies führt dazu, dass an den Bruchstellen der gebrochenen Rippen auch weiterhin reibende Bewegungen der Bruchteile aneinander auftreten, die Schmerzen verursachen. Diese Schmerzen können zu einer Verkrampfung der intercostalen Muskulatur führen, wodurch die Schmerzen weiter verstärkt werden.

### DARSTELLUNG DER ERFINDUNG

Es ist daher Aufgabe der Erfindung, eine schmerzlindernde Immobilisierungsvorrichtung für die Anwendung bei Thoraxfrakturen zu schaffen, welche die Nachteile bekannter Vorrichtungen vermeidet und insbesondere einfach aufgebaut und herzustellen ist, auf einfache Weise und besonders sicher angewendet werden kann und beim Patienten zu einer Linderung der Schmerzen und Verbesserung der Atmung führt, ohne ihn in seiner Bewegungsfreiheit wesentlich zu behindern.

Die Aufgabe wird durch die Gesamtheit der Merkmale des Anspruchs 1 gelöst. Der Kern der Erfindung besteht darin, ein in sich steifes, flächiges Schienungselement vorzusehen, welches den Bruchbereich und ggf. die gebrochene(n) Rippe(n) und benachbarte ungebrochene Rippen überdeckt, und es auf der dem Körper zugewandten Seite mit einer zum Aufkleben der Immobilisierungsvorrichtung auf den Körper geeigneten Klebeschicht zu versehen. Das Schienungselement kann dann auf den Bereich des Thorax geklebt werden, in welchem die Fraktur lokalisiert ist (Bruchbereich), wobei vorzugsweise benachbarte, unversehrte Bereiche mit überdeckt werden. Das in sich vergleichsweise steife Schienungselement kann so die gebrochenen Rippen selbst verschienen und sich ggf. an den gesunden Rippen abstützen. Diese Stabilisierung führt zu einer Verminderung der Schmerzen und einer Erleichterung der Atmung.

Gemäss einer bevorzugten Ausgestaltung der Erfindung ist das Schienungselement, insbesondere ohne zusätzliche Hilfsmittel oder Werkzeuge, an die äussere Kontur des Thorax anpassbar, wobei es vorzugsweise eine plastisch verformbare Kunststoffplatte oder ein plastisch verformbares Metallblech umfasst. Hierdurch wird die Wirksamkeit der Schienung weiter erhöht und die Anwendung weiter vereinfacht.

Vorzugsweise besteht das plastisch verformbare Metallblech aus Aluminium, wobei das plastisch verformbare Metallblech zur Verbesserung der lokalen Verformbarkeit bei gleichzeitiger Erhöhung der Steifigkeit gewellt ausgebildet ist, und die Wellenkämme sich im wesentlichen parallel zu den zu behandelnden Rippen erstrecken. Ein derartiges Schienenmaterial ist bereits in anderem Zusammenhang mit grossem Erfolg eingesetzt worden (siehe die WO-A1-97/22312 bzw die US-A-6,039,706).

Die Bequemlichkeit beim Tragen des Schienungselements kann dadurch verbessert werden, dass das Schienungselement auf der Ober- und/oder Unterseite mit einer Abdeckung versehen ist, wobei die obere und/oder untere Abdeckung vorzugsweise aus einem Gewebe oder elastischen, insbesondere offenporigen, Schaumstoff besteht. Zusätzlich können in dem Schienungselement Durchbrechungen wie z.B. Löcher verteilt angeordnet sein, um eine Durchlässigkeit der immobilisierungsvorrichtung zu erreichen.

Damit die Immobilisierungsvorrichtung gegen äussere Einflüsse wie Wasser oder dgl. geschützt ist, ist es von Vorteil, wenn eine Schutzfolie zur Bedeckung der Oberseite des Schienungselements vorgesehen ist. Die Schutzfolie kann insbesondere nach Anbringung des Schienungselements am Körper flächig über das Schienungselement geklebt werden. Ein seitlicher Schutz wird dabei auf einfache Weise dadurch erreicht, dass die Schutzfolie über das Schienungselement seitlich übersteht und einen umlaufenden Randstreifen bildet, und dass die Schutzfolie auf der Unterseite auch im Bereich des Randstreifens mit einer Klebeschicht versehen ist.

Um die mit einer Rippenfraktur verbundenen Schmerzen weiter abzusenken, ist es vorteilhaft, wenn die Immobilisierungsvorrichtung zusätzlich lokale Mittel zur Linderung von Schmerzen umfasst. Dazu können die schmerzlindernden Mittel in einem mit der Immobilisierungsvorrichtung lösbar verbindbaren Kissen oder Pad untergebracht sein. Es ist aber auch denkbar, dass Teilbereiche der Klebeschicht oder die ganze Klebeschicht mit einem schmerzlindernden Mittel versehen sind.

### KURZE ERLÄUTERUNG DER FIGUREN

Die Erfindung soll nachfolgend anhand von Ausführungsbeispielen im Zusammenhang mit der Zeichnung näher erläutert werden. Es zeigen
- Fig. 1: in einer stark vereinfachten perspektivischen Darstellung eine Immobilisierungsvorrichtung zur Ruhigstellung verletzter Rippen gemäss einem ersten Ausführungsbeispiel der Erfindung;
- Fig. 2: die Immobilisierungsvorrichtung aus Fig. 1 in der Draufsicht von oben;
- Fig. 3: In der Draufsicht von vorne eine beispielhafte Rippenfraktur, wobei vier Rippen dargestellt sind, von denen die zweitoberste eine Bruchstelle aufweist;
- Fig. 4: den vereinfachten Schnitt durch die Rippenfraktur aus Fig. 3 entlang der Linie IV-IV mit dem Bruchbereich;
- Fig. 5: In der Draufsicht von vorne die auf die Rippenfraktur aus Fig. 3 aufgeklebte Immobilisierungsvorrichtung gemäss einem zweiten Ausführungsbeispiel der Erfindung;
- Fig. 6: die Wirkung der aufgeklebten Immobilisierungsvorrichtung in einer zu Fig. 4 vergleichbaren Darstellung; und
- Fig. 7: den Schnitt durch die Immobilisierungsvorrichtung aus Fig. 5 und 6 in einer vergrösserten Darstellung.

### WEGE ZUR AUSFÜHRUNG DER ERFINDUNG

Die erfindungsgemässe Vorrichtung findet ihre Anwendung bei Rippenbrüchen (Thoraxfrakturen) oder -prellungen. In diesen Fällen geht es darum, dass die Bewegung der verletzten Rippen im Brustkorb verhindert oder zumindest stark eingeschränkt wird. Insbesondere ist es von Vorteil, dass mit der Vorrichtung im Falle eines Fensterbruches (bei dem mehrere Rippen jeweils an zwei voneinander beabstandeten Stellen gebrochen sind und so eine Art Fenster bilden) die auftretenden Paradoxatmung positiv beeinflusst werden kann.

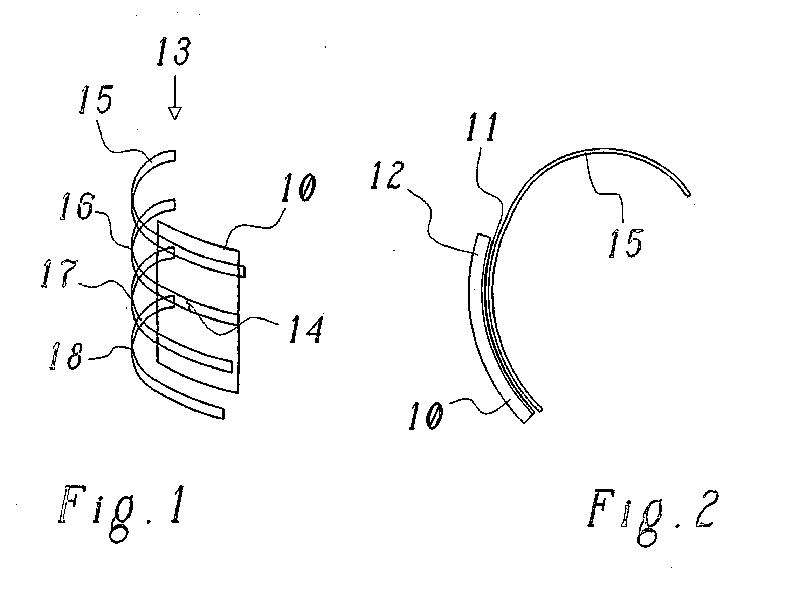

Ein Ausführungsbeispiel für eine derartige Immobilisierungsvorrichtung und deren Anwendung ist in Fig. 1 und 2 stark vereinfacht dargestellt. In Fig. 1 sind schematisiert vier Rippen 15,..,18 von einer Seite eines Brustkorbes 13 gezeigt, von denen die zweite Rippe von oben, die Rippe 16, eine Bruchstelle 14 aufweist. Die über den Rippen 15,..,18 liegenden Gewebe- und Hautschichten des Körpers sind der Einfachheit halber weggelassen. Ebenso ist die Zwischenrippenmuskulatur (Intercostalmuskulatur) nicht gezeigt. Auf den Bereich des Brustkorbes 13, der die Bruchstelle 14 umgibt, ist nun von aussen der Wölbung des Brustkorbes 13 angepasst eine flächige schienenartige Immobilisierungsvorrichtung 10 grossflächig oder ganzflächig aufgeklebt. Hauptbestandteil der Immobilisierungsvorrichtung 10 ist ein Schienungselement 12 (Fig. 2) in Form einer Platte oder eines Bleches aus einem in sich hinreichend steifen, in der Form aber gleichwohl plastisch veränderbaren Material. Die Klebung erfolgt, ähnlich wie bei einem Pflaster, durch eine auf der Innenseite des Schienungselements 12 angebrachten, geeignete Klebeschicht 11 (Fig. 2). Die Grösse (laterale Ausdehnung) der Immobilisierungsvorrichtung 10 ist dabei vorzugsweise so gewählt, dass die Immobilisierungsvorrichtung 10 nicht nur die verletzte Rippe 16, sondern auch die benachbarten Rippen 15 und 17 in ausreichender Weise überdeckt.

Durch die Klebung stützt sich die Immobilisierungsvorrichtung 10 an dem unverletzten Teil der gebrochenen Rippe(n) und den benachbarten unverletzten Rippen 15 und 17 ab und fixiert die verletzte Rippe 16 selbst und in ihrer räumlichen Lage relativ zu den benachbarten Rippen 15 und 17. Dadurch wird die schmerzende Bewegung der verletzten Rippe 16 beim Atmen, Husten, Lachen oder anderweitiger körperlicher Bewegung stark eingeschränkt und der mit der Bewegung verbundene Schmerz verhindert oder zumindest reduziert.

Zusätzlich können auf der Innenseite der Immobilisierungsvorrichtung 10 lokal Mittel zur Linderung der durch die verletzte Rippe 16 verursachten Schmerzen vorgesehen werden. Denkbar ist dabei insbesondere ein mit einem schmerzlindernden, durch die Haut einwirkenden, Mittel getränktes Kissen oder Pad, das auf der Innenseite der Immobilisierungsvorrichtung 10, z.B. durch aufkleben oder mittels eines Klettbandes, lösbar befestigt wird. Es ist aber auch denkbar, Teilbereiche der Klebeschicht 11 oder die ganze Klebeschicht 11 mit einem entsprechenden schmerzlindernden Mittel zu versehen.

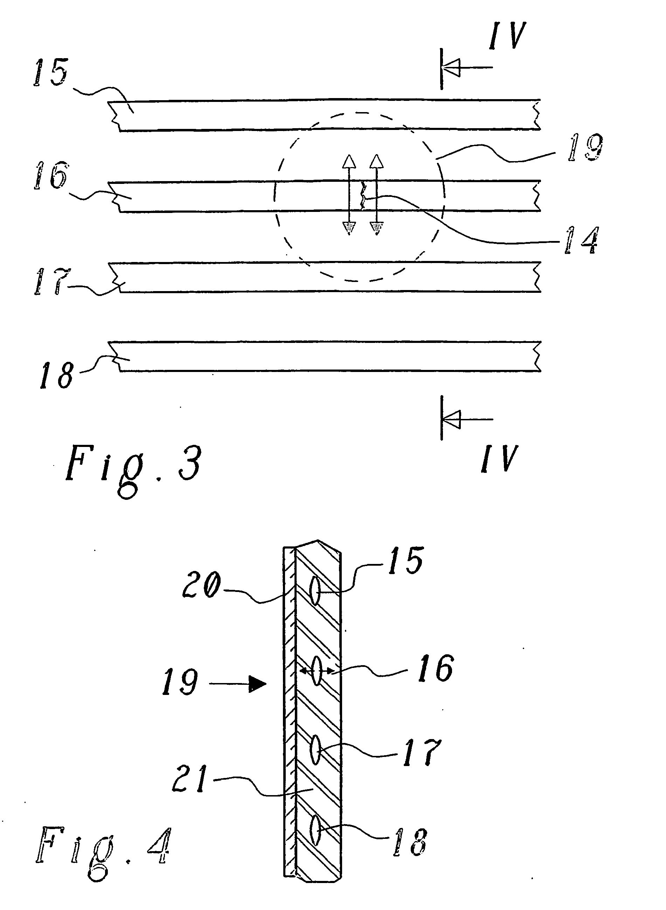

Der Effekt der erfindungsgemässen Immobilisierungsvorrichtung lässt sich anhand der Fig. 3 bis 6 erläutern: Ausgegangen wird auch in diesem Beispiel von vier parallelen Rippen 15,..,18, von denen die zweite von oben, die Rippe 16, eine Bruchstelle oder Fraktur 14 aufweist (selbstverständlich können auch mehrere Rippen gebrochen sein). Betrachtet man den Schnitt durch den Brustkorb entlang der Ebene IV-IV in Fig. 3, ergibt sich in vereinfachter Form die in Fig. 4 dargestellte Konfiguration. Die Rippen 15,.., 18 sind in die unter anderem zum Atmen benötigte Zwischenrippenmuskulatur 21 eingebettet. Darüber befindet sich eine mehrlagige Schicht aus Haut und Fettgewebe, die vereinfacht als eine Haut/Fettgewebeschicht 20 eingezeichnet ist. Im Bereich der Fraktur (Bruchbereich 19) verliert nun die gebrochene Rippe 16 zumindest teilweise ihre Stabilität und es kommt zu einer (in Fig. 3 und 4 durch Doppelpfeile angedeuteten) reibenden Bewegung der Bruchenden relativ zueinander, die dem Patienten bei jeder Bewegung des Brustkorbes erhebliche Schmerzen bereitet.

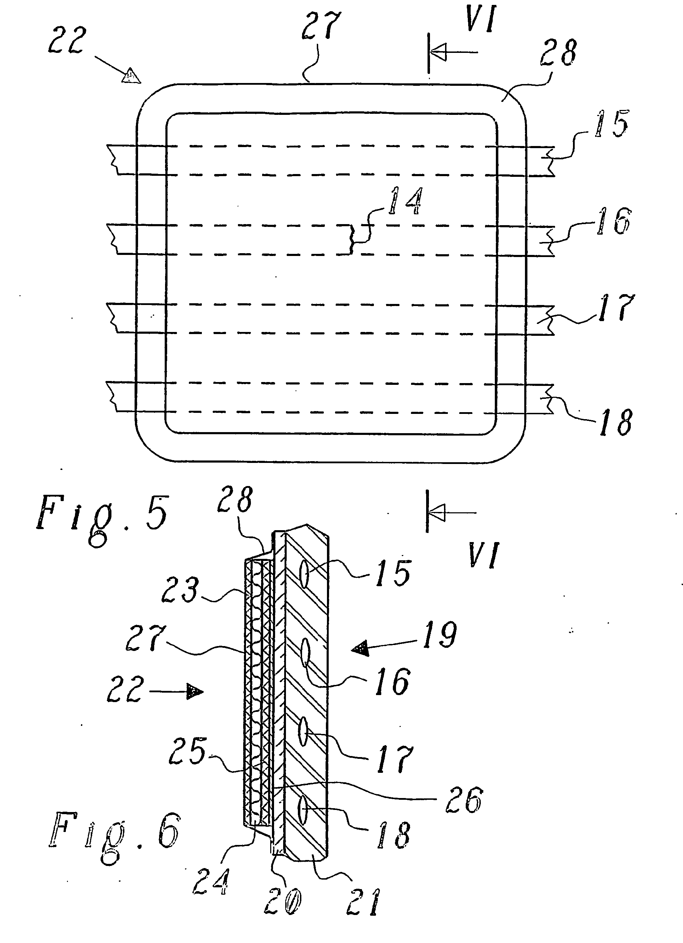

Wird nun gemäss Fig. 5 und 6 eine flächige Immobilisierungsvorrichtung 22 über den Bruchbereich 19 mit der gebrochenen Rippe 16 und vorzugsweise auch die benachbarten, nicht gebrochenen Rippen 15, 17 und 18 geklebt, wird der Bruchbereich 19 fixiert, indem die gebrochene Rippe 16 sowohl in sich als auch relativ zu den anderen Rippen 15, 17 und 18 immobilisiert wird. Dies führt dazu, dass insbesondere die Atmung des Patienten mit deutlich weniger Schmerzen verbunden ist und so wesentlich verbessert werden kann.

In klinischen Versuchen wurde die Schmerzintensität bei 42 Patienten (33 mit der erfindungsgemässen Immobilisierungsvorrichtung, 9 Kontrollpatienten), die Frakturen von bis zu 5 benachbarten Rippen aufwiesen, mittels visueller Analogskala vor sowie 1-2, 24 bzw. 48 Stunden nach Aufnahme in die Untersuchung bestimmt. Verglichen mit den Kontrollpatienten war die Schmerzintensität in Ruhe (p < 0,05), vor allem jedoch bei forcierter Inspiration (p < 0,01) über die gesamte Zeit signifikant vermindert. Die Schmerzlinderung durch die Immobilisierungsvorrichtung 10 bzw. 22 war bereits eine Stunde nach der Anlegung messbar, während bei den Kontrollpatienten erst nach 2-3 Tagen eine Abnahme der Schmerzintensität gemessen werden konnte.

Bei 18 Patienten wurden spirometrische Messungen vor dem Anlegen (Aufkleben) sowie 1-2, 24 und 48 Stunden (bei einigen Patienten zu jedem dieser Zeitpunkte) nach dem Anlegen der Immobilisierungsvorrichtung durchgeführt. Je nach Ausdehnung des Frakturareals kamen zwei Grössen der Immobilisierungsvorrichtung (12 cm x 17 cm und 15 cm x 18 cm) zur Anwendung. Bei fünf weiteren Patienten (Kontrollpatienten) wurde der Frakturbereich lediglich durch Operationstücher abgedeckt. Bei diesen Kontrollpatienten nahm die durch die Frakturen bereits eingeschränkte forcierte Vitalkapazität (FVC) nach 1-2 Stunden um im Mittel 174 ml weiter ab, und war auch nach 24 bzw. 48 Stunden mit plus 4 bzw. 34 ml kaum verbessert. Im Gegensatz hierzu verbesserte sich die FVC bei den mit der Immobilisierungsvorrichtung behandelten Patienten kontinuierlich und hoch signifikant (p < 0,001) um im Mittel 153 ml nach 1-2 Stunden, sowie um 384 bzw. 474 ml nach 24 bzw. 48 Stunden Anwendung der Immobilisierungsvorrichtung. Wie die FVC verbesserten sich auch die spirometrischen Parameter FEV1, IVC und PEF durch die Immobilisierungsvorrichtung.

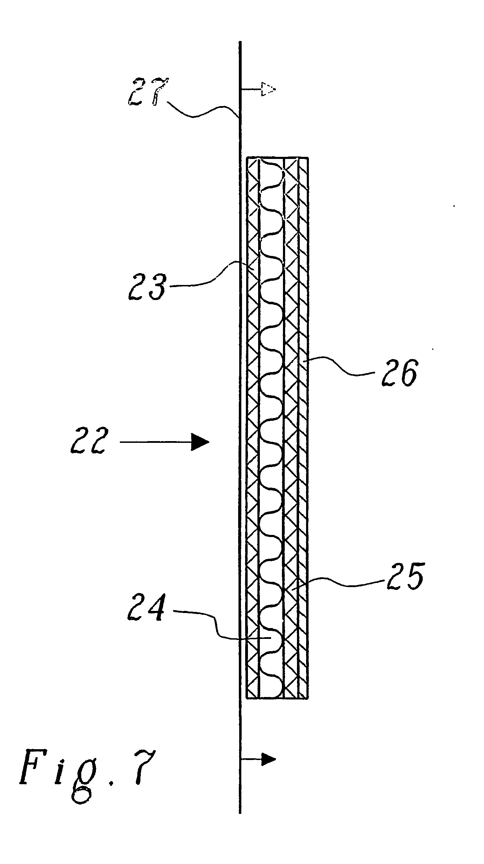

Eine bevorzugte Ausführungsform der Immobilisierungsvorrichtung 22 ist in Fig. 5-7 wiedergegeben. Die Immobilisierungsvorrichtung 22 umfast als zentrales Element ein flächiges Schienungselement 24, das im vorliegenden Beispiel aus einem gewellten Aluminiumblech besteht. Dicke des Bleches und Wellung sind so gewählt, dass das Schienungselement 24 einerseits mit den blossen Händen und ohne weitere Hilfsmittel leicht an die Wölbung des Brustkorbs im Bereich der zu behandelnden Fraktur angepasst werden kann, und andererseits ausreichend steif ist, um die Immobilisierungs- und Stützfunktion für die Fraktur sicher zu erfüllen. Bewährt haben sich Schienungselemente, wie sie in der WO-A1-97/22312 beschrieben worden sind.

Damit die Immobilisierungsvorrichtung 22 dem Brustkorb optimal angepasst werden kann, verlaufen die Wellenkämme des Schienungselements 24 parallel zu den Rippen. Das Schienungselement 24 ist zur Verbesserung des Tragekomforts auf der Ober- und Unterseite mit einer Abdeckung 23 bzw. 25 versehen. Die Abdeckungen 23, 25 bestehen vorzugsweise aus einem elastischen, geschäumten, offenporigen oder perforierten Kunststoffmaterial. Die untere Abdeckung 25 ist auf ihrer Aussenseite mit einer Klebeschicht 26 versehen, mit der die Immobilisierungsvorrichtung 22 auf den Frakturbereich geklebt werden kann. Als Kleber für die Klebeschicht kommen solche Kleber in Betracht, die sich für medizinische Anwendungen als geeignet erwiesen haben.

Die Oberseite der Immobilisierungsvorrichtung 22, d.h., die Aussenseite der oberen Abdeckung 23, wird bei der Anwendung mit einer Schutzfolie 27 beklebt, die seitlich übersteht und einen umlaufenden Rand 28 bildet (Fig. 5). Wird die Schutzfolie 27 mit dem überstehenden Rand 28 auf die Haut des Patienten geklebt, ist die Immobilisierungsvorrichtung 22 gegen äussere Einflüsse geschützt, so dass der Patient ohne negative Folgen beispielsweise duschen kann. Die Schutzfolie ist insbesondere atmungsaktiv und wasserdicht. Anstelle des gewellten Aluminiumbleches können für das Schienungselement 24 im Rahmen der Erfindung auch andere Materialien wie Kunststoffplatten oder dgl. eingesetzt werden, sofern sie über die nötige Steifigkeit verfügen und zugleich ausreichend plastisch verformbar sind. Das Schienungselement 24 ist vorzugsweise mit Durchbrechungen, z.B. in Form einer Perforation, versehen, um durchlässig zu sein und den Tragekomfort zu erhöhen.

### BEZUGSZEICHENLISTE

- 10,22: Immobilisierungsvorrichtung
- 11: Klebeschicht
- 12: Schienungselement (flächig)
- 13: Brustkorb
- 14: Bruchstelle
- 15,..,18: Rippe
- 19: Bruchbereich
- 20: Haut/Fettgewebe
- 21: Zwischenrippenmuskulatur
- 23: obere Abdeckung
- 24: Schienungselement (flächig)
- 25: untere Abdeckung
- 26: Klebeschicht
- 27: Schutzfolie
- 28: Rand (Schutzfolie)

## Patentansprüche

1. Vorrichtung (10, 22) zur schmerzlindernden Immobilisierung bei Thorax- bzw. Rippenfrakturen, die ein flächiges Schienungselement (12, 24) umfasst, welches den Bruchbereich (19) grossflächig überdeckt, wobei die Immobilisierungsvorrichtung (10, 22) auf der dem Körper zugewandten Seite eine zum Aufkleben der Immobilisierungsvorrichtung (10, 22) auf den Körper geeignete Klebeschicht (11, 26) aufweist, **dadurch gekennzeichnet, dass** das Schienungselement (12, 24) in sich steif ist.

2. Immobilisierungsvorrichtung nach Anspruch 1, **dadurch gekennzeichnet, dass** das Schienungselement (12, 24) ohne zusätzliche Hilfsmittel oder Werkzeuge an die äussere Kontur des Thorax anpassbar ist.

3. Immobilisierungsvorrichtung nach Anspruch 1 oder 2, **dadurch gekennzeichnet, dass** das Schienungselement (12, 24) eine plastisch verformbare Kunststoffplatte umfasst.

4. Immobilisierungsvorrichtung nach Anspruch 1 oder 2, **dadurch gekennzeichnet, dass** das Schienungselement (12, 24) ein plastisch verformbares Metallblech umfasst.

5. Immobilisierungsvorrichtung nach Anspruch 4, **dadurch gekennzeichnet, dass** das plastisch verformbare Metallblech aus Aluminium besteht.

6. Immobilisierungsvorrichtung nach Anspruch 5, **dadurch gekennzeichnet, dass** das plastisch verformbare Metallblech zur Verbesserung der lokalen Verformbarkeit bei gleichzeitiger Erhöhung der Steifigkeit gewellt ausgebildet ist, und dass die Wellenkämme sich im wesentlichen parallel zu den zu behandelnden Rippen erstrecken.

7. Immobilisierungsvorrichtung nach einem der Ansprüche 1 bis 6, **dadurch gekennzeichnet, dass** das Schienungselement (12, 24) auf der Ober-und/oder Unterseite mit einer Abdeckung (23, 25) versehen ist.

8. Immobilisierungsvorrichtung nach Anspruch 7, **dadurch gekennzeichnet, dass** die obere und/oder untere Abdeckung (23, 25) aus einem Gewebe oder einem elastischen, vorzugsweise offenporigen, Schaumstoff besteht.

9. Immobilisierungsvorrichtung nach einem der Ansprüche 1 bis 8, **dadurch gekennzeichnet, dass** zur schützenden Abdeckung der Oberseite des Schienungselements (12, 24) eine Schutzfolie (27) vorgesehen ist.

10. Immobilisierungsvorrichtung nach Anspruch 9, **dadurch gekennzeichnet, dass** die Schutzfolie (27) über das Schienungselement (12, 24) seitlich überstehend ausgebildet ist und einen umlaufenden Randstreifen (28) bildet, und dass die Schutzfolie (27) auf der Unterseite mit einer Klebeschicht versehen ist.

11. Immobilisierungsvorrichtung nach Anspruch 10, **dadurch gekennzeichnet, dass** die Immobilisierungsvorrichtung (10, 22) zusätzlich lokale Mittel zur Linderung von Schmerzen umfasst.

12. Immobilisierungsvorrichtung nach Anspruch 11, **dadurch gekennzeichnet, dass** die schmerzlindernden Mittel in einem mit der Immobilisierungsvorrichtung (10, 22) lösbar verbindbaren Kissen oder Pad untergebracht sind.

13. Immobilisierungsvorrichtung nach Anspruch 11, **dadurch gekennzeichnet, dass** Teilbereiche der Klebeschicht (11, 26) oder die ganze Klebeschicht (11, 26) mit einem schmerzlindernden Mittel versehen sind.

14. Immobilisierungsvorrichtung nach Anspruch 1, **dadurch gekennzeichnet, dass** das Schienungselement (12, 24) mit einer Perforation versehen ist.

## Claims

1. Device (10, 22) for analgesic immobilization for use in cases of thorax resp. rib fractures, the immobilizing device (10, 22) comprises a flat splint element (12, 24) overlapping a large surface at the fracture area (19) and the side of immobilizing device (10, 22) facing the body is provided with an appropriate adhesive layer (11, 26) for adhering the immobilizing device (10, 22) to the body **characterized in that** the splint element (12, 24) is rigid in itself.

2. Immobilizing device according to claim 1 **characterized in that** the splint element (12, 24) can be fitted on the outside contour of the thorax without any additional aid or tool.

3. Immobilizing device according to claim 1 or 2 **characterized in that** the splint element (12, 24) consists of a plastically deformable plastic plate.

4. Immobilizing device according to claim 1 or 2 **characterized in that** the splint element (12, 24) consists of a plastically deformable metal plate.

5. Immobilizing device according to claim 4 **characterized in that** the plastically deformable metal plate is made of aluminium.

6. Immobilizing device according to claim 5 **characterized in that** the plastically deformable metal plate is corrugated in order to improve the local deformability enlarging at the same time the rigidity, where the crests of corrugations run essentially parallel to the ribs to be treated.

7. Immobilizing device according to any of claims 1-6 **characterized in that** the splint element (12, 24) is provided with a covering (23, 25) on its top and/or bottom surface.

8. Immobilizing device according to claim 7 **characterized in that** the covering (23, 25) at the top and/or bottom surface consists of a tissue or of an elastic, preferably open-pored foam.

9. Immobilizing device according to any of claims 1-8 **characterized in that** it is provided with a protecting foil (27) for covering and protecting the top side of the splint element (12, 24).

10. Immobilizing device according to claim 9 **characterized in that** the protecting foil (27) covering said splint element (12, 24) is designed to extend laterally beyond the sides forming thereby a peripheral edgestrip (28), and that the protecting foil (27) is provided with an adhesive layer on its bottom side.

11. Immobilizing device according to claim 10 **characterized in that** the immobilizing device (10, 22) comprises also a local analgesic agent.

12. Immobilizing device according to claim 11 **characterized in that** the analgesic agent is contained in a pad or cushion contacted to the immobilizing device (10, 22) via a releasable bond.

13. Immobilizing device according to claim 11 **characterized in that** parts of the adhesive layer (11, 26) or the whole adhesive layer (11, 26) is provided with an analgesic agent.

14. Immobilizing device according to claim 1 **characterized in that** the spint element (12, 24) is provided with a perforation.

## Revendications

1. Dispositif (10, 22) pour l'immobilisation analgésique dans le cas des fractures de thorax resp. de côtes, comprenant une éclisse (12, 24) plate recouvrant une grande surface dans la région de la zone fracturée (19), où le côté du dispositif d'immobilisation (10, 22) faisant face au corps est équipé d'une couche adhésive (11, 26) appropriée pour coller le dispositif d'immobilisation (10, 22) au corps, **caractérisé en ce que** l'éclisse (12, 24) en soi est rigide.

2. Dispositif d'immobilisation selon la revendication 1, **caractérisé en ce que** l'éclisse (12, 24) peut être adaptée sur le contour extérieur du thorax sans aide ou outil supplémentaire.

3. Dispositif d'immobilisation selon la revendication 1 ou 2, **caractérisé en ce que** l'éclisse (12, 24) comprend une plaque en matière plastique qui est plastiquement déformable.

4. Dispositif d'immobilisation selon la revendication 1 ou 2, **caractérisé en ce que** l'éclisse (12, 24) comprend une plaque métallique qui est plastiquement déformable.

5. Dispositif d'immobilisation selon la revendication 4, **caractérisé en ce que** la plaque métallique plastiquement déformable est faite de l'aluminium.

6. Dispositif d'immobilisation selon la revendication 5, **caractérisé en ce que** la plaque métallique plastiquement déformable est ondulée afin d'améliorer la déformabilité locale, en augmentant en même temps la rigidité, où les crêtes des ondulations dans la plaque sont essentiellement parallèles aux côtes à traiter.

7. Dispositif d'immobilisation selon l'une quelconque des revendications 1-6, **caractérisé en ce que** l'éclisse (12, 24) est équipée d'une couverture (23, 25) sur sa surface supérieure et/ou inférieure.

8. Dispositif d'immobilisation selon la revendication 7, **caractérisé en ce que** la couverture (23, 25) supérieure et/ou inférieure se compose d'un tissu ou d'une mousse élastique, de préférence avec pores ouverts.

9. Dispositif d'immobilisation selon l'une quelconque des revendications 1-8, **caractérisé en ce qu'**on l'équipe d'un film protecteur (27) pour la couverture protectrice du côté supérieur de l'éclisse (12, 24).

10. Dispositif d'immobilisation selon la revendication 9, **caractérisé en ce que** le film protecteur (27) sur l'éclisse (12, 24) est développé de sorte que sa forme soit plus grande aux côtés formant de ce fait une bande de rebord (28) circulaire, et le film protecteur (27) est équipé d'une couche adhésive de son côté inférieur.

11. Dispositif d'immobilisation selon la revendication 10, **caractérisé en ce que** le dispositif d'immobilisation (10, 22) contient également un agent analgésique local.

12. Dispositif d'immobilisation selon la revendication 11, **caractérisé en ce que** l'agent analgésique est contenu dans un coussin ou pad attaché détachable au dispositif d'immobilisation (10, 22).

13. Dispositif d'immobilisation selon la revendication 11, **caractérisé en ce que** parts de la couche adhésive (11, 26) ou la couche adhésive entière (11, 26) sont équipées d'un agent analgésique.

14. Dispositif d'immobilisation selon la revendication 1, **caractérisé en ce que** l'éclisse (12, 24) est équipée d'une perforation.
